# EUROPEAN PATENT APPLICATION

(11) **EP 4 647 059 A2**
(43) Date of publication of application: **12.11.2025**
(21) Application number: 25162464.9
(22) Date of filing: 07.03.2025
(51) Int. Cl.: A61K 8/02, A61K 8/25, A61K 8/31, A61K 8/44, A61K 8/73, A61Q 19/10

(54) **POWDER GRANULES, AND COSMETIC PREPARATION AND CLEANSING PREPARATION**

(30) Priority: 21.03.2024 JP 2024045095
(71) Applicant: Shiseido Honeycake Industries Co., Ltd., Ibaraki-shi, Osaka 567-0021 (JP)
(72) Inventor: NISHINA, Tetsuo, Osaka, 567-0021 (JP); SAITO, Yoshinobu, Osaka, 567-0021 (JP); OGAWA, Tai, Osaka, 567-0021 (JP); HARADA, Koki, Osaka, 567-0021 (JP)
(74) Representative: Henkel & Partner mbB

(57) **Abstract**

Provided are powder granules containing (A) a powder component and (C) ethylcellulose, wherein the powder component (A) is (A-1) an amino acid derivative, which is lauroyl lysine or caproyl lysine, and wherein a content of the amino acid derivative (A-1) is 80 to 95% by mass, and a content of the ethylcellulose (C) is 1 to 10% by mass.

## Description

### CROSS-REFERRENCE TO RELATED APPLICATIONS

The present application claims priority from Japanese Patent Application No. 2024-45095, filed March 21, 2024, the disclosure of which is incorporated herein by reference in its entirety.

### TECHNICAL FIELD

The present invention relates to novel powder granules, and a cosmetic preparation and a cleansing preparation containing the powder granules.

### BACKGROUND ART

Powder granules can be blended into various cleansing agents to impart various functions and effects to the products. The functions and effects of powder granules are that a characteristic appearance and a scrubbing effect can be imparted, and that various foam-quality improvers or chemical agents that are difficult to be directly blended into cleansing agents can be blended in the form of powder granules.

However, when powder granules are blended into a cleansing agent, the powder granules float or settle in the cleansing agent during preparation due to the difference in specific gravity between the cleansing agent and the powder granules, and thus it has been difficult to stably blend powder granules into a cleansing agent.

Thus, powder granules have been proposed which contain a polyethylene powder, a binder, and a liquid additive, so as to facilitate adjusting the specific gravity of the powder granules relative to that of the liquid component of the cleansing agent (see, for example,

Patent Literature 1).

However, plastic fine particles such as polyethylene powder, when discharged into sewage, may not be completely collected in a sewage treatment plant and may flow into rivers and lakes. The surface of these plastic fine particles is readily compatible with harmful substances, and may accumulate in the bodies of fishes that have eaten the plastic fine particles.

Therefore, there is an increasing concern, mainly in the United States, that plastic fine particles may flow into rivers or lakes to pollute the environment.

It is also known to substitute the plastic fine particles with a powder obtained by grinding plant seeds. However, the particles of this powder are not round and hard, and thus have the drawback of being highly irritating to the skin or mucosa.

It is also known that volcanic ash, cellulose or calcium carbonate can be used. However, since these materials have high specific gravity, adjusting their specific gravity is very difficult, these materials have the drawback that the granules settle with time.

Furthermore, the use of a cleansing agent containing a volcanic ash-derived insoluble component has the drawback of being too irritating to the eyes, if the volcanic ash-derived insoluble component enters the eyes.

Thus, the present applicant conducted extensive research and as a result, previously proposed powder granules containing (A) 25 to 75% by mass of a silicone powder with a specific gravity of 0.8 to 1.0; (B) 20 to 70% by mass of a cellulose powder; and (C) 1 to 5% by mass of ethylcellulose (see Patent Literature 2). The powder granules proposed therein have excellent characteristics, i.e., the powder granules are less irritating to the skin, and the specific gravity of the powder granules can be easily adjusted to be equivalent to that of, for example, a liquid component of a cleansing preparation, that of a liquid, emulsion, gel, or cream preparation, or that of liquid soap before cooling and solidification of a soap manufactured by the frame process.

### Citation List

### Patent Literature

[Patent Literature 1] Japanese Unexamined Patent Publication No. 2001-207196
[Patent Literature 2] Japanese Patent No. 6074492

### SUMMARY OF THE INVENTION

### TECHNICAL PROBLEM

However, the silicone powder contained in the powder granules disclosed in Patent Literature 2 is not biodegradable and remains in the environment, and thus the use of the silicone powder may be limited.

The present invention has been made in view of the above-mentioned circumstances, and it is an object of the present invention to provide novel powder granules that have sufficient strength during granulation and storage, and undergo a reduction in strength when used by being blended into a liquid, emulsion, gel, or cream preparation, or a framed soap to easily dissolve and disintegrate, and are satisfactory in granulation properties, stability in tank, and appearance when blended into a soap and also satisfactory in disintegrating properties when blended into the preparation. It is also an object of the present invention to provide a cosmetic preparation and a cleansing preparation.

### SOLUTION TO PROBLEM

The present inventors have conductive extensive research to solve the above-mentioned problem and as a result, found that novel powder granules can be obtained by using (A-1) an amino acid derivative, which is lauroyl lysine or caproyl lysine, and/or (A-2) a wax having a melting point of 100°C or more, as new base, and by adding (C) ethylcellulose as a binder and preferably (B) talc, thus completing the present invention.

The present invention is based on the above-mentioned findings by the present inventors, and the solution to the above-mentioned problem is as set forth below.

First powder granules according to the present invention contain (A) a powder component; and (C) ethylcellulose, wherein the powder component (A) is (A-1) an amino acid derivative, which is lauroyl lysine or caproyl lysine, and wherein a content of the amino acid derivative (A-1) is 80 to 95% by mass, and a content of the ethylcellulose (C) is 1 to 10% by mass.

Second powder granules according to the present invention contain (A) powder components; and (C) ethylcellulose, wherein the powder components (A) are (A-1) an amino acid derivative, which is lauroyl lysine or caproyl lysine, and (A-2) a wax having a melting point of 100°C or more, and wherein a content of the amino acid derivative (A-1) is 85 to 95% by mass, a content of the wax (A-2) having a melting point of 100°C or more is 1 to 10% by mass, and a content of the ethylcellulose (C) is 1 to 10% by mass.

Third powder granules according to the present invention contain (A) a powder component; (B) talc; and (C) ethylcellulose, wherein the powder component (A) is (A-2) a wax having a melting point of 100°C or more, wherein a content of the wax (A-2) having a melting point of 100°C or more is 75 to 90% by mass, a content of the talc (B) is 5 to 20% by mass, and a content of the ethylcellulose (C) is 1 to 10% by mass, wherein a mass ratio of the wax (A-2) having a melting point of 100°C or more to the talc (B) [(A-2)/(B)] is 3 to 20, and wherein the powder granules have a theoretical specific gravity of 0.9 to 1.1.

The first powder granules preferably further contain cellulose.

In the first powder granules, a content of the cellulose is preferably 5 to 15% by mass.

In the first powder granules, the lauroyl lysine (A-1) preferably has an average particle diameter of 10 to 20 µm.

The first powder granules preferably have a melting point of 90°C or more.

The first powder granules preferably have a theoretical specific gravity of 1.1 to 1.2, more preferably 1.197 to 1.211.

In the second powder granules, the wax (A-2) having a melting point of 100°C or more preferably has an average particle diameter of 1 to 30 µm.

In the second powder granules, the wax (A-2) having a melting point of 100°C or more is preferably a synthetic wax.

In the second powder granules, the lauroyl lysine (A-1) preferably has an average particle diameter of 10 to 20 µm.

The second powder granules preferably have a melting point of 90°C or more.

The second powder granules preferably have a theoretical specific gravity of 1.1 to 1.2, more preferably 1.165 to 1.181.

In the third powder granules, the wax (A-2) having a melting point of 100°C or more is preferably a synthetic wax.

The third powder granules preferably have a melting point of 90°C or more.

A cleansing preparation of the present invention contains the powder granules according to any one of the first to third aspects of the present invention.

A cosmetic preparation of the present invention contains the powder granules according to any one of the first to third aspects of the present invention.

### ADVANTAGEOUS EFFECTS OF THE INVENTION

According to the present invention, it is possible to overcome the above-mentioned drawbacks in the prior art to achieve the above-mentioned object, and to provide novel powder granules that have sufficient strength during granulation and storage, and undergo a reduction in strength when used by being blended into a liquid, emulsion, gel, or cream preparation, or a framed soap to easily dissolve and disintegrate, and are satisfactory in granulation properties, stability in tank, and appearance when blended into a soap and also satisfactory in disintegrating properties when blended into the preparation. According to the present invention, it is also possible to provide a cosmetic preparation and a cleansing preparation.

### DETAILED DESCRIPTION OF THE INVENTION

### (Powder Granules According to First Aspect)

Powder granules according to a first aspect of the present invention contain (A) a powder component and (C) ethylcellulose, and optionally further contain other components.

In the first powder granules, the powder component (A) is (A-1) an amino acid derivative, which is lauroyl lysine or caproyl lysine; and a content of the amino acid derivative (A-1) is 80 to 95% by mass, and a content of the ethylcellulose (C) is 1 to 10% by mass.

### (Powder Granules According to Second Aspect)

Powder granules according to a second aspect of the present invention contain (A) powder components and (C) ethylcellulose, and optionally further contain other components.

In the second powder granules, the powder components (A) are (A-1) an amino acid derivative, which is lauroyl lysine or caproyl lysine, and (A-2) a wax having a melting point of 100°C or more; and a content of the amino acid derivative (A-1) is 85 to 95% by mass, a content of the wax (A-2) having a melting point of 100°C or more is 1 to 10% by mass, and a content of the ethylcellulose (C) is 1 to 10% by mass.

### (Powder Granules According to Third Aspect)

Powder granules according to a third aspect of the present invention contain (A) a powder component, (B) talc, and (C) ethylcellulose, and optionally further contain other components.

In the third powder granules, the powder component (A) is (A-2) a wax having a melting point of 100°C or more; a content of the wax (A-2) having a melting point of 100°C or more is 75 to 90% by mass, a content of the talc (B) is 5 to 20% by mass, and a content of the ethylcellulose (C) is 1 to 10% by mass; and a mass ratio of the wax (A-2) having a melting point of 100°C or more to the talc (B) [(A-2)/(B)] is 3 to 20.

The third powder granules have a theoretical specific gravity of 0.9 to 1.1, preferably 0.981 to 1.095. It should be noted here that the theoretical specific gravity can be calculated based on equation 1 below, wherein when the powder granules are composed of components A, B, C, ... N; A', B', C', ... N' represent the contents of the respective components, and a, b, c, ... n represent the specific gravities of the respective components. (Theoretical Specific Gravity) = 100/{(A'/a) + (B'/b) + (C'/c) + ... + (N'/n)} with the proviso that A' + B' + C' + **...** N' = 100; and a, b, c, ... n > 0.

The powder granules according to any one of the first to third aspects of the present invention are less irritating to the skin, and the specific gravity of the powder granules can be easily adjusted to be equivalent to that of a liquid component of a cleansing preparation, that of a liquid, emulsion, gel, or cream preparation, or that of liquid soap before cooling and solidification of a soap manufactured by the frame process.

Furthermore, the powder granules according to any one of the first to third aspects of the present invention have sufficient strength during granulation and storage, and undergo a reduction in strength when used by being blended into a liquid, emulsion, gel, or cream preparation, or a framed soap to easily dissolve and disintegrate, and are satisfactory in granulation properties, stability in tank, and appearance when blended into a soap and also satisfactory in disintegrating properties when blended into the preparation.

### <Powder Component (A)>

The powder component (A) is at least one of (A-1) an amino acid derivative, which is lauroyl lysine or caproyl lysine, and (A-2) a wax having a melting point of 100°C or more.

When the amino acid derivative (A-1), which is lauroyl lysine or caproyl lysine, is used as the powder component (A), the amino acid derivative (A-1), which is lauroyl lysine or caproyl lysine, may be used alone, or the amino acid derivative (A-1) and the wax (A-2) having a melting point of 100°C or more may be used in combination.

When the wax (A-2) having a melting point of 100°C or more is used as the powder component (A), the wax (A-2) having a melting point of 100°C or more may be used alone, or the amino acid derivative (A-1), which is lauroyl lysine or caproyl lysine, and the wax (A-2) having a melting point of 100°C or more may be used in combination.

When the wax (A-2) having a melting point of 100°C or more is used alone as the powder component (A), the powder granules preferably further contain the talc (B).

### - Amino Acid Derivative (A-1) -

The amino acid derivative (A-1) is a compound obtained by dehydration condensation of a higher fatty acid and an amino acid, and examples include lauroyl lysine and caproyl lysine. Among these, lauroyl lysine is particularly preferred in terms of the granulation properties.

Lauroyl lysine is an amide of lauric acid and lysine, represented by the following structural formula:

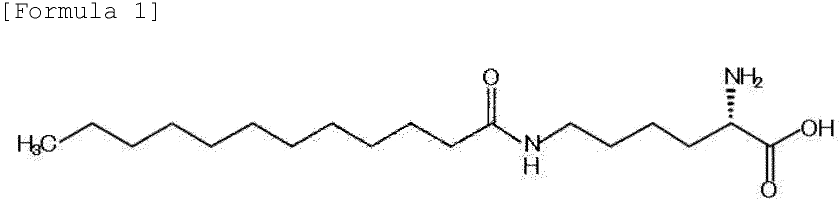

Lauroyl lysine is a layered powder with a structure in which hexagonal, plate-like thin crystals are layered. Lauroyl lysine preferably has an average particle diameter of preferably 30 µm or less, more preferably 20 to 30 µm.

Examples of commercially available lauroyl lysine include AMIHOPE (R) LL (available from Ajinomoto Co., Inc).

The content of the amino acid derivative (A-1) in the powder granules is preferably 80% by mass or more, and more preferably 80 to 95% by mass. If the content of the amino acid derivative in the powder granules is less than 80% by mass, the powder granules can be granulated but may be slightly sticky when gently held by hand, and the powder granules when blended into a soap may dissolve or aggregate in the soap, and slightly float or settle.

Furthermore, when manufacturing powder granules using the amino acid derivative (A-1) in combination with the wax (A-2) having a melting point of 100°C or more as the powder components (A), the content of the amino acid derivative in the powder granules is preferably 85 to 95% by mass, in view of the appearance when blended into a soap. When the wax (A-2) having a melting point of 100°C or more is used in combination, if the content of the amino acid derivative in the powder granules is less than 85% by mass, the powder granules when blended into a soap may dissolve or aggregate in the soap, and may slightly float or settle. On the other hand, if the content of the amino acid derivative is more than 95% by mass and the content of the ethylcellulose (C) as a binder is less than 1% by mass, the binding effect may be weak, and the powder granules may lose its stability (may easily disintegrate).

When the wax (A-2) having a melting point of 100°C or more is used as the powder component (A), the wax (A-2) is preferably used in combination with the amino acid derivative (A-1) or talc (B), in view of the granulation properties, the stability in tank, and the appearance when blended into a soap.

### - Wax (A-2) Having a Melting Point of 100°C or More as Powder Component -

The wax (A-2) having a melting point of 100°C or more is not specifically limited as long as it is a wax having a melting point of 100°C or more, but is preferably a synthetic wax.

Examples of synthetic waxes include hydrocarbon waxes (for example, paraffin wax and microcrystalline wax) .

The synthetic wax has an average particle diameter of preferably 1 to 30 µm, more preferably 10 to 30 µm.

When the wax (A-2) having a melting point of 100°C or more is used in combination with the talc (B), the content of the wax having a melting point of 100°C or more in the powder granules is preferably 75 to 90% by mass, in view of the granulation properties, the stability in tank, and the appearance when blended into a soap. When the talc (B) is used in combination, if the content of the wax (A-2) having a melting point of 100°C or more in the powder granules is less than 75% by mass, the powder granules when blended into a soap may dissolve or aggregate in the soap, and may significantly float or settle. On the other hand, if the content of the wax (A-2) having a melting point of 100°C or more is more than 90% by mass and the content of the ethylcellulose (C) as a binder is less than 1% by mass, the binding effect may be weak, and the powder granules may lose its stability (may easily disintegrate).

Furthermore, when manufacturing powder granules using the amino acid derivative (A-1) and the wax (A-2) having a melting point of 100°C or more in combination as the powder components (A), the content of the wax (A-2) having a melting point of 100°C or more in the powder granules is preferably 1 to 10% by mass, in view of the appearance when blended into a soap. When the wax (A-2) having a melting point of 100°C or more is used in combination with the amino acid derivative (A-1), if the content of the wax (A-2) having a melting point of 100°C or more is more than 10% by mass, the powder granules when blended into a soap may dissolve or aggregate in the soap, and may slightly float or settle.

### <Talc (B)>

Examples of inorganic powders used in the present invention include talc, sericite, mica, titanium mica, titanium oxide, kaolin, and silicates. These inorganic powders may be used alone or in combinations of two or more. Among these, the talc (B) is particularly preferred in view of achieving a satisfactory feel when the powder granules are held by hand.

The talc (B) has an average particle diameter of preferably 1 to 30 µm, more preferably 1 to 10 µm.

The talc (B) is used in combination with the wax (A-2) having a melting point of 100°C or more.

When the wax (A-2) having a melting point of 100°C or more as the powder component (A) and the talc (B) are used in combination, the content of the talc (B) in the powder granules is preferably 5 to 20% by mass, in view of the granulation properties, the stability in tank, and the appearance when blended into a soap. When the talc (B) is used in combination with the wax (A-2) having a melting point of 100°C or more, if the content of the talc (B) is less than 5% by mass, the specific gravity of the powder granules may be reduced, and the granules may float when blended into a soap. On the other hand, if the content of the talc (B) is more than 20% by mass, the powder granules when blended into a soap may dissolve or aggregate in the soap, and may significantly float or settle.

The mass ratio of the wax (A-2) having a melting point of 100°C or more to the talc (B) [(A-2)/(B)] is 3 to 20, and preferably 3.8 to 18.

### <Ethylcellulose (C)>

Examples of the binder used in the present invention include ethylcellulose, vinyl acetate resins, gum arabic, xanthan gum, and carrageenan. These binders may be used alone or in combinations of two or more. Among these, the ethylcellulose (C) is particularly preferred because it has a satisfactory color and odor, and has excellent granulation properties.

The content of the ethylcellulose (C) is preferably 1 to 10% by mass in the powder granules, in terms of binding properties and granulation properties.

The powder granules of the present invention preferably further contain cellulose. The content of the cellulose is preferably 5 to 15% by mass in the powder granules.

### <Other Components>

The powder granules of the present invention may optionally contain other components as long as they do not interfere with the object and effects of the present invention.

Examples of other components include colorants, synthetic chemical agents, oils, antioxidants, light stabilizers, ultraviolet absorbents, thermal stabilizers, tackifiers, and processing aids.

While the particle diameter of the powder granules of the present invention is not specifically limited, the powder granules of the present invention have an average particle diameter of preferably 100 to 1,000 µm, more preferably 200 to 500 µm.

A soap composition containing the powder granules of the present invention is usually treated at 70 to 90°C during the preparation. Thus, the powder granules have a melting point of preferably 90°C or more, more preferably 95°C or more.

### <Method for Manufacturing Powder Granules>

Granulation methods for the powder granules of the present invention include, but are not specifically limited to, agitation mixing granulation, tumbling granulation, extrusion granulation, crushing granulation, fluidized bed granulation, spray drying granulation, melt granulation, compression granulation, vacuum freeze granulation, flocculated suspension granulation, and coating granulation. Among these, extrusion granulation is preferred.

To manufacture the powder granules of the present invention, the components (A) and (C) or the components (A) to (C), and optionally cellulose or other components, are charged into an agitation apparatus, a solvent is added in three portions, and the contents are stirred each time the solvent is added to obtain a mixture. The resulting mixture is granulated by extruding through a sieve with a predetermined opening to obtain granules. The resulting granules are dried by evaporating the solvent. Then, the granules are subjected to extrusion granulation through a sieve with a predetermined opening. In this way, powder granules can be manufactured. The resulting powder granules may optionally be further subjected to size classification or pulverization to adjust the particle diameter.

Examples of the solvent include ethanol, methanol, acetone, and hexane. Among these, ethanol is preferred in view of safety.

### (Cosmetic Preparation)

A cosmetic preparation of the present invention contains the powder granules of the present invention.

The content of the powder granules is preferably 0.1 to 10% by mass, more preferably 0.5 to 5% by mass, still more preferably 0.5 to 3% by mass, and particularly preferably 1 to 3% by mass, based on the total amount of the cosmetic preparation.

A base of the cosmetic preparation containing the powder granules may be appropriately selected from components usually used in cosmetic preparations, and examples include water, alcohols, oily materials, surfactants, moisturizers, whitening agents, thickeners, pH adjusters, UV absorbents, antioxidants, preservatives, sequestrants (chelating agents), coloring materials, perfumes, excipients, blood circulation promoters, chemical agents for skin, chemical agents for scalp, other medicinal agents, vitamins, hormones, amino acids, and antihistamines.

Examples of dosage forms of the cosmetic preparation include creams, pastes, gels, liquids, and powders.

Specific products of the cosmetic preparation include skin lotions, milky lotions, serums, creams, gels, cleansing foams, cleansing powders, cleansing creams, massage creams, massaging gels, hair treatments, hair masks, hair conditioners, lotions, and sheet masks.

### (Cleansing Preparation)

A cleansing preparation of the present invention contains the powder granules of the present invention.

The content of the powder granules is preferably 0.1 to 10% by mass, more preferably 0.5 to 5% by mass, still more preferably 0.5 to 3% by mass, and particularly preferably 1 to 3% by mass, based on the total amount of the cleansing preparation.

A base of the cleansing preparation containing the powder granules may be appropriately selected from components usually used in cleansing preparations, and examples include higher fatty acid salts, acyl glutamates, cetyl sulfate, polyoxyalkylene alkylamines, cationic polymers, thickeners, chelating agents, pH adjusters, perfumes, colorants, moisturizers, preservatives, antiinflammatory agents, plant extracts, bactericides, antioxidants, UV absorbents, chelating agents, higher fatty acids, and sequestrants.

Examples of dosage forms of the cleansing preparation include solids, liquids, emulsions, creams, pastes, and gels.

Specific products of the cleansing preparation include framed soaps, transparent soaps, solid shampoos, conditioning shampoos, body soaps, and hand soaps.

### Examples

Examples of the present invention will be hereinafter described; however, the present invention is not limited to these examples. Evaluation methods used in the present invention are as follows.

### (1) Granulation Properties

The granulation properties represent the texture of the resulting powder granules when held by hand and whether the resulting powder granules are capable of being granulated. The granulation properties were evaluated based on the following criteria:

### [Evaluation Criteria]

A: The powder granules contained an appropriate amount of moisture, were capable of maintaining the shape for a while when gently held by hand, were not sticky and capable of being uniformly granulated.
B: The powder granules were not sticky when gently held by hand, but were capable of being granulated.
C: The powder granules were sticky when gently held by hand, but were capable of being granulated.
D: The powder granules were not capable of being granulated.

### (2) Stability in Tank during Manufacture

The stability in tank during manufacture represents whether or not the powder granules dissolve and/or disintegrate when stirred for several minutes in the soap composition shown in Table 1 below. The stability in tank during manufacture was evaluated based on the following criteria:

### [Evaluation Criteria]

A: The powder granules were added into a soap-making tank (70°C) and stirred; after 15 minutes of stirring, the powder granules neither dissolved nor disintegrated.
B: The powder granules were added into a soap-making tank (70°C) and stirred; after 15 minutes of stirring, the powder granules partially dissolved and disintegrated.
C: The powder granules were added into a soap-making tank (70°C) and stirred; after 15 minutes of stirring,
the powder granules completely dissolved and disintegrated.

### (3) Appearance

The appearance when the powder granules were blended into the soap composition shown in Table 1 below was evaluated based on the following criteria:

### [Evaluation Criteria]

A: The powder granules were uniformly dispersed throughout the soap composition, without floating or settling.
B: The powder granules dissolved or aggregated in the soap composition, and slightly floated or settled.
C: The powder granules dissolved or aggregated in the soap composition, and significantly floated or settled.

### (4) Theoretical Specific Gravity

The theoretical specific gravity was calculated based on equation 1 below, wherein when the powder granules are composed of components A, B, C, ... N; A', B', C', ... N' represent the contents of the respective components, and a, b, c, ... n represent the specific gravities of the respective components. (Theoretical Specific Gravity) = 100/{(A'/a) + (B'/b) + (C'/c) + ... + (N'/n)} with the proviso that A' + B' + C' + ... N' = 100; and a, b, c, ... n > 0.

### - Soap Composition -

**[Table 1]**

| Components | Content (% by Mass) |
|---|---|
| Powder Granules | 0.2 |
| Lauric Acid | 5.0 |
| Myristic Acid | 11.0 |
| Palmitic Acid | 3.0 |
| Stearic Acid | 5.0 |
| Isostearic Acid | 2.0 |
| Potassium Hydroxide | 1.0 |
| Sodium Hydroxide | 4.0 |
| PPG-8 Glyceryl | 5.0 |
| Glycerin | 16.0 |
| Sucrose | 12.0 |
| Sorbitol | 2.0 |
| Sodium Lauryl Glycol Carboxylate | 1.5 |
| Sodium Lauroamphoacetate | 1.0 |
| PEG-30 Hydrogenated Castor Oil | 8.0 |
| Salt | QS |
| Chelating Agent | QS |
| Ethanol | 5.0 |
| Ion-Exchanged Water | Balance |
| Total (% by mass) | 100.0 |

### (Test Examples 1-1 to 1-9)

First, powders that could substitute for a silicone powder were investigated.

Each powder shown in Table 2 was used and charged into an agitation apparatus together with lauroyl lysine and ethylcellulose, a solvent (ethanol) was added in three portions, and the contents were stirred each time the solvent was added to obtain a mixture.

The resulting mixture was granulated by extruding through a 20 mesh (20 M) sieve to obtain granules.

The resulting granules were dried by evaporating the solvent.

Then, the granules were granulated by extruding through a 60 mesh (60 M) sieve to obtain powder granules.

The 20 mesh (20 M) sieve is a sieve with an opening of 0.701 mm (701 µm).

The 60 mesh (60 M) sieve is a sieve with an opening of 0.246 mm (246 µm).

Next, the granulation properties of the resulting powder granules were evaluated. The results are shown in Table 2.

Moreover, the stability in tank during manufacture was evaluated for the soap composition shown in Table 1 above containing the resulting powder granules, and overall evaluation was determined. The results are shown in Table 2.

**[Table 2]**

| Test Example | Component Name | Average Particle Diameter (µm) | Specific Gravity | Melting Point (°C) | Granulation Properties | Stabil ity in Tank | Overall Evaluat ion |
|---|---|---|---|---|---|---|---|
| 1-1 | Polyethylene Powder | 20 | 0.92 | - | - | - | - |
| 1-2 | Polysilicone-22 | 20 | 0.95 | - | - | - | - |
| 1-3 | Lauroyl Lysine | 10~20 | 1.20 | 200 | A | A | A |
| 1-4 | Batyl Alcohol | - | 0.99 | 65 | D | - | C |
| 1-5 | Calcium Stearate | 14 | 1.08 | 148 | D | - | C |
| 1-6 | Cellulose | 10 | 1.30 | 170 | C | B | B |
| 1-7 | Carnauba Wax | 20 | 0.69 | 85 | A | C | C |
| 1-8 | Hydrogenated Castor/Jojoba Esters | 7 | 0.98 | 85 | A | C | C |
| 1-9 | Synthetic Wax | 7 | 0.94 | 111 | A | A | A |

The investigation results in Table 2 show that lauroyl lysine (Test Example 1-3) and the synthetic wax (Test Example 1-9), which have excellent granulation properties and excellent stability in tank during manufacture, are suitable for the powder granules.

### (Test Examples 2-1 to 2-7)

Next, cellulose was used as a base, and a suitable content of lauroyl lysine in the powder granules was investigated.

According to Table 3, powder granules were produced as in Test Examples 1-1 to 1-9, and the granulation properties of the resulting powder granules were evaluated. The results are shown in Table 3.

Moreover, for the soap composition shown in Table 1 above containing the resulting powder granules, the stability in tank during manufacture and the appearance when blended were evaluated. The results are shown in Table 3.

**[Table 3]**

| Component Name | | Specific Gravity | Test Example | | | | | | |
|---|---|---|---|---|---|---|---|---|---|
| | | | 2-1 | 2-2 | 2-3 | 2-4 | 2-5 | 2-6 | 2-7 |
| Base | Cellulose | 1.30 | 95 | 75 | 55 | 35 | 15 | 5 | - |
| | Lauroyl Lysine | 1.20 | - | 20 | 40 | 60 | 80 | 90 | 95 |
| Binder | Ethylcellulose | 1.14 | 5 | 5 | 5 | 5 | 5 | 5 | 5 |
| Total (% by Mass) | | - | 100 | 100 | 100 | 100 | 100 | 100 | 100 |
| Theoretical Specific Gravity | | - | 1.291 | 1.270 | 1.250 | 1.230 | 1.211 | 1.201 | 1.197 |
| Solvent | Ethanol | - | 25 | 25 | 25 | 25 | 25 | 25 | 25 |
| Granulation Properties | | - | C | C | B | B | A | A | A |
| Stability in Tank during Manufacture | | - | B | B | B | A | A | A | A |
| Appearance When Blended | | - | C | C | B | B | A | A | A |

The results in Table 3 show that when the content of lauroyl lysine in the powder granules is 40% by mass or more (Test Examples 2-3 and 2-4), the granulation properties and the appearance (settling) when blended into a soap tend to improve, and when the content of lauroyl lysine in the powder granules is 80% by mass or more (Test Examples 2-5 and 2-7), the granulation properties, the stability in tank, and the appearance when blended are satisfactory.

### (Test Examples 3-1 to 3-12)

Next, cellulose was used as a base, and a suitable content of the synthetic wax in the powder granules was investigated.

According to Tables 4-1 and 4-2, powder granules were produced as in Test Examples 1-1 to 1-9, and the granulation properties of the resulting powder granules were evaluated. The results are shown in Tables 4-1 and 4-2.

Moreover, for the soap composition shown in Table 1 above containing the resulting powder granules, the stability in tank during manufacture and the appearance when blended were evaluated. The results are shown in Tables 4-1 and 4-2.

**[Table 4-1]**

| Component Name | | Specific Gravity | Test Example | | | | | |
|---|---|---|---|---|---|---|---|---|
| | | | 3-1 | 3-2 | 3-3 | 3-4 | 3-5 | 3-6 |
| Base | Cellulose | 1.30 | 95 | 75 | 55 | 35 | 15 | 5 |
| | Synthetic Wax | 0.94 | - | 20 | 40 | 60 | 80 | 90 |
| | Talc | 2.80 | - | - | - | - | - | - |
| Binder | Ethylcellulose | 1.14 | 5 | 5 | 5 | 5 | 5 | 5 |
| Total (% by Mass) | | - | 100 | 100 | 100 | 100 | 100 | 100 |
| Theoretical Specific Gravity | | - | 1.291 | 1.200 | 1.120 | 1.051 | 0.990 | 0.962 |
| Solvent | Ethanol | - | 25 | 25 | 25 | 25 | 25 | 25 |
| Granulation Properties | | - | C | C | B | B | A | A |
| Stability in Tank during Manufacture | | - | B | B | A | A | A | A |
| Appearance When Blended | | - | C | C | C | C | C | C |

**[Table 4-2]**

| Component Name | | Specific Gravity | Test Example | | | | | |
|---|---|---|---|---|---|---|---|---|
| | | | 3-7 | 3-8 | 3-9 | 3-10 | 3-11 | 3-12 |
| Base | Cellulose | 1.30 | - | - | - | - | - | - |
| | Synthetic Wax | 0.94 | 95 | 90 | 85 | 75 | 70 | 65 |
| | Talc | 2.80 | - | 5 | 10 | 20 | 25 | 30 |
| Binder | Ethylcellulose | 1.14 | 5 | 5 | 5 | 5 | 5 | 5 |
| Total (% by Mass) | | - | 100 | 100 | 100 | 100 | 100 | 100 |
| Theoretical Specific Gravity | | - | 0.948 | 0.981 | 1.016 | 1.095 | 1.139 | 1.187 |
| Solvent | Ethanol | - | 25 | 25 | 25 | 25 | 25 | 25 |
| Granulation Properties | | - | A | A | A | A | A | A |
| Stability in Tank during Manufacture | | - | A | A | A | A | A | A |
| Appearance When Blended | | - | B | A | A | A | C | C |

The results in Tables 4-1 and 4-2 show that when the content of the synthetic wax in the powder granules is 80% by mass or more (Test Examples 3-5 to 3-7), the granulation properties tend to improve, but the soap composition tends to be slightly turbid when the powder granules are blended into the soap composition.

The results also show that when the synthetic wax is used in combination with talc, the granulation properties, the stability in tank, and the appearance when blended improve (Test Examples 3-7 to 3-10), and when the content of the synthetic wax is 75 to 90% by mass and the content of talc is 5 to 20% by mass in the powder granules, the powder granules had good granulation properties, stability in tank, and appearance when blended (Test Examples 3-8 to 3-10).

### (Test Example 2-7 and Test Examples 4-1 to 4-10)

Next, the effect of using lauroyl lysine and the synthetic wax in combination was investigated.

According to Tables 5-1 and 5-2, powder granules were produced as in Test Examples 1-1 to 1-9, and the granulation properties of the resulting powder granules were evaluated. The results are shown in Tables 5-1 and 5-2.

Moreover, for the soap composition shown in Table 1 above containing the resulting powder granules, the stability in tank during manufacture and the appearance when blended were evaluated. The results are shown in Tables 5-1 and 5-2.

**[Table 5-1]**

| Component Name | | Specific Gravity | Test Example | | | | | |
|---|---|---|---|---|---|---|---|---|
| | | | 2-7 | 4-1 | 4-2 | 4-3 | 4-4 | 4-5 |
| Base | Lauroyl Lysine | 1.20 | 95 | 90 | 85 | 80 | 75 | 70 |
| | Synthetic Wax | 0.94 | - | 5 | 10 | 15 | 20 | 25 |
| Binder | Ethylcellulose | 1.14 | 5 | 5 | 5 | 5 | 5 | 5 |
| Total (% by Mass) | | - | 100 | 100 | 100 | 100 | 100 | 100 |
| Theoretical Specific Gravity | | - | 1.197 | 1.181 | 1.165 | 1.149 | 1.134 | 1.120 |
| Solvent | Ethanol | - | 25 | 25 | 25 | 25 | 25 | 25 |
| Granulation Properties | | - | A | A | A | A | A | A |
| Stability in Tank during Manufacture | | - | A | A | A | A | A | A |
| Appearance When Blended | | - | A | A | A | B | C | C |

**[Table 5-2]**

| Component Name | | Specific Gravity | Test Example | | | | |
|---|---|---|---|---|---|---|---|
| | | | 4-6 | 4-7 | 4-8 | 4-9 | 4-10 |
| Base | Lauroyl Lysine | 1.20 | 50 | 30 | 10 | 5 | - |
| | Synthetic Wax | 0.94 | 45 | 65 | 80 | 90 | 95 |
| Binder | Ethylcellulose | 1.14 | 5 | 5 | 5 | 5 | 5 |
| Total (% by Mass) | | - | 100 | 100 | 95 | 100 | 100 |
| Theoretical Specific Gravity | | - | 1.065 | 1.015 | 1.022 | 0.959 | 0.948 |
| Solvent | Ethanol | - | 25 | 25 | 25 | 25 | 25 |
| Granulation Properties | | - | A | A | A | A | A |
| Stability in Tank during Manufacture | | - | A | A | A | A | A |
| Appearance When Blended | | - | C | C | C | C | C |

The results in Tables 5-1 and 5-2 confirm that when lauroyl lysine and the synthetic wax are used in combination, and when the content of the synthetic wax in the powder granules is 15% by mass or more (Test Examples 4-3 to 4-10), the appearance when blended into a soap tends to deteriorate.

It was found that the powder granules had good granulation properties, stability in tank, and appearance (Test Examples 2-7, 4-1, and 4-2) when the content of lauroyl lysine is 85 to 95% by mass and the content of the synthetic wax is 1 to 10% by mass in the powder granules in the case where lauroyl lysine and the synthetic wax are used in combination.

### (Formulation Examples 1 to 3)

Table 6 below shows Formulation Examples 1 to 3 of soap compositions containing the powder granules of the present invention.

**[Table 6]**

| Components(% by mass) | Formulation Example | | |
|---|---|---|---|
| | 1 | 2 | 3 |
| Powder Granules of Test Example 2-7 | 0.2 | - | - |
| Powder Granules of Test Example 3-9 | - | 0.2 | - |
| Powder Granules of Test Example 4-2 | - | - | 0.2 |
| Lauric Acid | 5.0 | 5.0 | 5.0 |
| Myristic Acid | 11.0 | 11.0 | 11.0 |
| Palmitic Acid | 3.0 | 3.0 | 3.0 |
| Stearic Acid | 5.0 | 5.0 | 5.0 |
| Isostearic Acid | 2.0 | 2.0 | 2.0 |
| Potassium Hydroxide | 1.0 | 1.0 | 1.0 |
| Sodium Hydroxide | 4.0 | 4.0 | 4.0 |
| PPG-8 Glyceryl | 5.0 | 5.0 | 5.0 |
| Glycerin | 16.0 | 16.0 | 16.0 |
| Sucrose | 12.0 | 12.0 | 12.0 |
| Sorbitol | 2.0 | 2.0 | 2.0 |
| Sodium Lauryl Glycol Carboxylate | 1.5 | 1.5 | 1.5 |
| Sodium Lauroamphoacetate | 1.0 | 1.0 | 1.0 |
| PEG-30 Hydrogenated Castor Oil | 8.0 | 8.0 | 8.0 |
| Salt | QS | QS | QS |
| Chelating Agent | QS | QS | QS |
| Ethanol | 5.0 | 5.0 | 5.0 |
| Ion-Exchanged Water | Balance | Balance | Balance |
| Total (% by mass) | 100.0 | 100.0 | 100.0 |

## Claims

1. Powder granules comprising:
(A) a powder component; and
(C) ethylcellulose,
wherein the powder component (A) is (A-1) an amino acid derivative, which is lauroyl lysine or caproyl lysine, and
wherein a content of the amino acid derivative (A-1) is 80 to 95% by mass, and a content of the ethylcellulose (C) is 1 to 10% by mass.

2. Powder granules comprising:
(A) powder components; and
(C) ethylcellulose,
wherein the powder components (A) are (A-1) an amino acid derivative, which is lauroyl lysine or caproyl lysine, and (A-2) a wax having a melting point of 100°C or more, and
wherein a content of the amino acid derivative (A-1) is 85 to 95% by mass, a content of the wax (A-2) having a melting point of 100°C or more is 1 to 10% by mass, and a content of the ethylcellulose (C) is 1 to 10% by mass.

3. Powder granules comprising:
(A) a powder component;
(B) talc; and
(C) ethylcellulose,
wherein the powder component (A) is (A-2) a wax having a melting point of 100°C or more, and
wherein a content of the wax (A-2) having a melting point of 100°C or more is 75 to 90% by mass, a content of the talc (B) is 5 to 20% by mass, and a content of the ethylcellulose (C) is 1 to 10% by mass,
wherein a mass ratio of the wax (A-2) having a melting point of 100°C or more to the talc (B) [(A-2)/(B)] is 3 to 20, and
wherein the powder granules have a theoretical specific gravity of 0.9 to 1.1.

4. The powder granules according to any one of claims 1 to 3, further comprising cellulose.

5. The powder granules according to claim 4, wherein a content of the cellulose is 5 to 15% by mass.

6. The powder granules according to claim 2 or 3, wherein the wax (A-2) having a melting point of 100°C or more has an average particle diameter of 1 to 30 µm.

7. The powder granules according to claim 2 or 3, wherein the wax (A-2) having a melting point of 100°C or more is a synthetic wax.

8. The powder granules according to claim 1 or 2, wherein the lauroyl lysine (A-1) has an average particle diameter of 10 to 20 µm.

9. The powder granules according to any one of claims 1 to 8, wherein the powder granules have a melting point of 90°C or more.

10. The powder granules according to claim 1 or 2, wherein the powder granules have a theoretical specific gravity of 1.1 to 1.2.

11. A cleansing preparation comprising the powder granules according to any one of claims 1 to 10.

12. A cosmetic preparation comprising the powder granules according to any one of claims 1 to 10.
